# EUROPÄISCHE PATENTANMELDUNG

(11) **EP 1 216 699 A1**
(43) Veröffentlichungstag der Anmeldung: **26.06.2002**
(21) Anmeldenummer: 00250449.6
(22) Anmeldetag: 21.12.2000
(51) Int. Cl.: A61K 9/70, A61K 31/575

(54) **Transdermalsystem enthaltend ein hochpotentes Gestagen**

(71) Anmelder: Schering Aktiengesellschaft, 13353 Berlin (DE)
(72) Erfinder: Lipp, Ralph, Priv.-Doz. Dr,, 14169 Berlin (DE); Günther, Clemens, Dr., 13357 Berlin (DE)

(57) **Zusammenfassung**

Transdermalsystem gekennzeichnet durch einen Gehalt an (21S)-21-Hydroxy-21-methyl-14,17-ethano-19-norpregna-4,9,15-trien-3,20-dion.

## Beschreibung

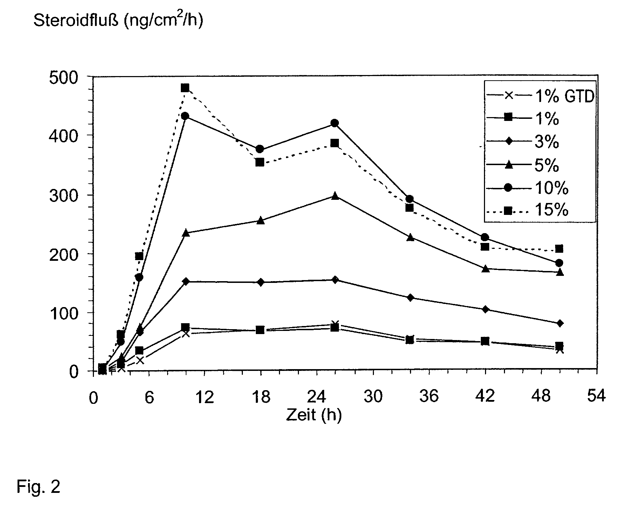

Die Erfindung betrifft ein Transdermalsystem (TDS) von hochpotenten Gestagen(en), speziell von (21S)-21-Hydroxy-21-methyl-14,17-ethano-19-norpregna-4,9,15-trien-3,20-dion, nachfolgend als Hydroxytriendion (Fig. 1) bezeichnet.

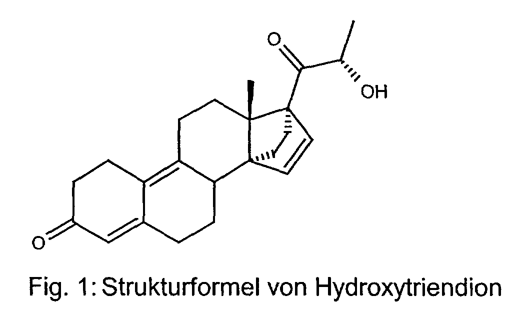

Eine besondere Form von transdermalen Formulierungen sind Transdermalsysteme.
Der Begriff "Transdermalsystem" wird im Sinne des Patentes in einer engen Definition für alle transdermalen Pflasterformulierungen verwendet. Üblicherweise werden Transdermalsysteme in Matrix-Transdermalsysteme und Membran-Transdermalsysteme unterteilt.

Matrix-Transdermalsysteme bestehen im einfachsten Fall aus drei parallel übereinander angeordneten Schichten, nämlich einer Rückschicht, einer Matrix und einer Abziehschicht. Letztere, die üblicherweise aus Kunststofffolie oder beschichtetem Papier besteht, wird vor der Applikation des Transdermalsystems auf die Haut entfernt. Die Matrix enthält den zu applizierenden Wirkstoff und hat üblicherweise gleichzeitig adhäsive Eigenschaften. Sollte die Matrix nicht adhäsiv genug sein, um zuverlässig an dem Hautbereich anzuhaften, auf den das Transdermalsystem appliziert werden soll, kann zwischen Matrix und Abziehschicht noch eine Kleberschicht vorgesehen werden.

Membran-Transdermalsysteme bestehen im einfachsten Fall aus vier Schichten, nämlich einer Rückschicht, einem Reservoir, einer Membran und einer Abziehschicht. Das Reservoir, das Wirk- und Hilfsstoffe enthalten kann, wird üblicherweise vollständig von Rückschicht und Membran umgeben. Die Inhaltsstoffe aus dem Reservoir können durch die Membran hindurch freigesetzt werden. Da eine Reihe von Membranen von sich aus nicht ausreichend adhäsiv sind, um an dem Hautbereich anzuhaften, auf den das Transdermalsystem appliziert werden soll, wird in der Regel zwischen Membran und Abziehschicht (zumindest im Randbereich) noch eine Kleberschicht vorgesehen.
Es ist bekannt, dass Gestagene transdermal appliziert werden können.
Die bisher in Formulierungen für transdermale Systeme eingesetzten Gestagene weisen jedoch in der Regel relativ niedrige Löslichkeiten in den verwendeten Matrizes auf, z.B. Gestoden oder Levonorgestrel ca. 1 %. Ein kristallfreies Transdermalsystem ist mit diesen Gestagenen nur herstellbar, wenn der Gehalt an Gestagen in der Matrix die Sättigungskonzentration nicht deutlich überschreitet.
Oft wird aber ein hoher Gehalt an Gestagen in der Matrix angestrebt, um ausreichende transdermalen Hautflüsse zu erzielen.

Andere Gestagene besitzen zwar eine höhere Löslichkeit, wie z. B. Norethistosteron (ca. 7 %), aber ihre gestagene Potenz ist vergleichsweise gering.

Der Erfindung liegt daher die Aufgabe zugrunde, ein Transdermalsystem mit einem hohen Gehalt an hoch potenten Gestagenen in gelöster Form zur Verfügung zu stellen.

Erfindungsgemäß wird diese Aufgabe durch ein Transdermalsystem mit einem Gehalt an dem hochpotenten Gestagen (21S)-21-Hydroxy-21-methyl-14,17-ethano-19-norpregna-4,9,15-trien-3,20-dion (Hydroxytriendion) gelöst.

Die Erfindung sieht in einer Ausführungsform vor, dass (21S)-21-Hydroxy-21-methyl-14,17-ethano-19-norpregna-4,9,15-trien-3,20-dion in einer Matrix, insbesondere einer Klebstoffmatrix vorliegt.

Als medizinisch akzeptable Kleber können beispielsweise Polyacrylat-, Silikonoder Polyisobutylen-Kleber eingesetzt werden. Darüber hinaus sind aber auch Polyurethane, Block-Copolymere auf Styrenbasis und weitere organische Polymere einsetzbar.

Bevorzugt sind Polyacrylatkleber.
Polyacrylat im Sinne des Patentes ist Oberbegriff für alle Polymere (Homo- und Copolymere), die Acrylsäure bzw. Acrylsäurederivate enthalten.
Besonders bevorzugt sind Vinylacetat-Acrylat-Copolymere und Acrylat-Vinylpyrrolidon-Copolymere. Am bevorzugtesten 2-Ethylhexylacrylat-Hydroxyethylacrylat-Copolymere (Gelva® ) sowie Copolymere der vorgenannten Verbindungen mit weiteren Substanzen wie beispielsweise Vinylacetat und 2-Ethylhexylacrylat-N-vinyl-2-pyrrolidon (TSR® -Kleber der Firma Sekisui)

Es ist ein Gehalt von 0,1 bis 20 Gew.-% Hydroxytriendion ((21S)-21-Hydroxy-21-methyl-14,17-ethano-19-norpregna-4,9,15-trien-3,20-dion), bevorzugt 1 bis 15 Gew.-%, in der Matrix vorgesehen.

In einer besonders bevorzugten Ausführungsform enthält die erfindungsgemäße transdermale Formulierung Kristallisationsinhibitoren, die als Komplexbildner geeignet sind, beispielsweise feste Lösungen mit Wirkstoffen zu bilden, die Grenzflächenlöslichkeit für den Wirkstoff erhöhen und die Neigung des Wirkstoffes zur Rekristallisation nach Entfernen eines Prozesslösungsmittels oder Senkung der Temperatur herabsetzen. Der Zusatz von Kristallisationsinhibitoren ermöglicht es, höhere Wirkstoffbeladungen der Formulierung vorzunehmen, ohne dass sich Wirkstoffkristalle bilden, die nur sehr eingeschränkt für den Stofftransport in die Haut zur Verfügung stehen. Als Kristallisationsinhibitoren sind N-Vinyllactam-Polymere wie N-Vinyl-1-Azacycloheptan-2-on-Homopolymer und N-Vinyl-piperidin-2-on-Homopolymer und insbesondere Polymere des Vinylpyrrolidons, wie Polyvidon (Kollidon® ), oder Co-Polymere des Vinylpyrrolidons mit Vinylacetat (Copovidone) geeignet. Besonders bevorzugt ist ein Copovidon aus 6 Teilen Vinylpyrrolidon und 4 Teilen Vinylacetat (Kollidon® VA 64).

Der Gehalt an Kristallisationsinhibitor im erfindungsgemäßen Transdermalsystem beträgt 0,1 bis 40%, vorzugsweise 2 bis 20%.

Das erfindungsgemäße System weist vorzugsweise einen zusätzlichen Gehalt an wenigstens einem Penetrationsverstärker auf.

Als Penetrationsverstärker können eingesetzt werden:
ein- oder mehrwertige Alkohole wie Ethanol, 1,2-Propandiol oder Benzylalkohol; gesättigte oder ungesättigte Fettalkohole mit 8 bis 18 Kohlenstoffatomen wie Laurylalkohol oder Cetylalkohol; Kohlenwasserstoffe wie Mineralöl; gesättigte und ungesättigte Fettsäuren mit 8 bis 18 Kohlenstoffatomen wie Stearinsäure oder Ölsäure; Fettsäureester mit bis zu 24 Kohlenstoffatomen oder Dicarbonsäurediester mit bis zu 24 Kohlenstoffatomen wie die Methylester, Ethylester, Isopropylester, Butylester, sec.-Butylester, Isobutylester, tert.-Butylester oder Monoglycerinsäureester der Essigsäure, Capronsäure, Laurinsäure, Myristinsäure, Stearinsäure und Palmitinsäure, Phosphatidderivate, wie Lecithin, Terpene, Harnstoff und seine Derivate oder Ether wie Dimethylisosorbid und Diethylenglycolmonoethylether.

Besonders bevorzugte sind Laurylalkohol, 1,2-Propandiol, die Methylester und insbesondere die Isopropylester der Myristinsäure oder Ölsäure, Diisopropyladipat und Diisopropylsebacat, Laurinsäure und Ölsäure, sowie deren Gemische.

Weiterhin können auch Mischungen aus einem oder mehreren penetrationsverstärkenden Mitteln für die erfindungsgemäße transdermal wirksame Formulierung verwendet werden. Hier werden Mischungen von bis zu vier Penetrationsverstärkem eingesetzt. Bevorzugt ist der Einsatz von binären und ternären Penetrationsverstärkergemischen. Am bevorzugtesten ist der Einsatz von binären Penetrationsverstärkergemischen aus hydrophilen mit lipophilen Penetrationsverstärkern eingesetzt.
Beispiele sind Enhancermischungen von Fettsäureestern oder Fettsäuren mit kurzkettigen, ein- oder zweiwertigen Alkoholen im Verhältnis 1:10 bis 10:1. Bevorzugtes Mischungsverhältnis ist 3:1 bis 1:3.

Der Gehalt an Penetrationsverstärker oder Penetrationsverstärkergemisch im erfindungsgemäßen Transdermalsystem beträgt 0,5 bis 40%, vorzugsweise 5 bis 25 %.

Weiterhin ist die Erfindung bevorzugt gekennzeichnet durch einen zusätzlichen Gehalt an wenigstens einem Estrogen.
Als Estrogen können eingesetzt werden: Estradiol, Estriol, Ethinylestradiol, deren Derivate wie beispielsweise Mono- und Di-ester wie das Estradiol-3,17ßdiproprionat.

Überraschenderweise wird mit (21S)-21-Hydroxy-21-methyl-14,17-ethano-19-norpregna-4,9,15-trien-3,20-dion (Hydroxytriendion) erstmals ein hoch potentes Gestagen zur Verfügung gestellt, das in Transdermalsystemen, insbesondere Matrix-Transdermalsystemen auf Polyacrylatkleber-Basis, eine überraschend hohe Löslichkeit von bis zu etwa 20 Gew.-% besitzt. Mittels solcher hochbeladener Systeme können außerordentlich hohe transdermale Flüsse erzielt werden.
Die Kombination von hoher gestagener Potenz, exzellenter Matrix-Beladbarkeit und Hautpenetration des Wirkstoffes ermöglicht es, unter Einsatz von Hydroxytriendion leistungsfähige Hormonersatz- oder Fertilitätskontroll-Transdermalsysteme zur Verfügung zu stellen.

Selbstverständlich ist es möglich, in die Matrix-Transdermalsysteme weitere Hilfsstoffe einzubeziehen, wie die oben erwähnten Kristallisationsinhibitoren und Penetrationsverstärker oder Lösungsvermittler und/oder Lösungsmittel.

Darüber hinaus können Penetrationsverstärker zur Erhöhung der Hautflüsse auch vor Applikation des Transdermalsystems auf die entsprechenden Hautpartien aufgetragen werden.

Die in der vorstehenden Beschreibung sowie in den Ansprüchen offenbarten Merkmale der Erfindung können sowohl einzeln als auch in beliebiger
Kombination für die Verwirklichung der Erfindung in ihren verschiedenen Ausführungsformen wesentlich sein

### Beispiele

### Beispiel 1: Herstellung von Transdermalsystemen mit Hydroxytriendion

Hydroxytriendion wird in ein Becherglas eingewogen und mit 25 g einer 30 %igen Lösung von Kollidon® VA 64 in 2-Propanol. Es werden 10 ml 2-Propanol zugesetzt. Die erhaltene Mischung wird 5 min. gerührt. Die Mischung wird in die vorgelegte Kleberlösung (Gelva® 7881) eingetragen. Die untenstehende Tabelle gibt die im vorgelegten Volumen der Kleberlösung enthaltene Klebertrockenmasse an. Anschließend wird weitere 0,5 h gerührt. Nach polarisationsmikroskopischer Prüfung auf Kristallfreiheit wird die erhaltene Mischung mittels einer 300 µm Rakel auf einen Liner aus fluorpolymerbeschichtetes Polyester (Scotchpak® 1022 Release Liner) gecoatet. Es wird 20 min. bei 70°C getrocknet und anschließend mit einem Backing aus einem Co-Laminat von PVC-PVDC mit Polyester (Saran-Hytrel® Backing) laminiert. Aus dem erhaltenen dreischichtigen Laminat werden runde Pflaster von 10 cm² Fläche gestanzt und in Siegelrandbeutel aus Aluminiumverbundfolie eingeschweißt.

| Formulierung | Gelva® 7881 | Hydroxytriendion Konz. im TDS (m/m%) | | Kollidon® VA 64 Konz. im TDS (m/m%) | |
|---|---|---|---|---|---|
| 1 | 42 g | 0,5 g | 1 | 7,5 g | 15 |
| 2 | 41 g | 1,5 g | 3 | 7,5 g | 15 |
| 3 | 40 g | 2,5 g | 5 | 7,5 g | 15 |
| 4 | 37,5 g | 5,0 g | 10 | 7,5 g | 15 |
| 5 | 35 g | 7,5 g | 15 | 7,5 g | 15 |
| Gelva® 7881 ist eine 50 %ige Polyacrylatkleber-Lösung in Ethylacetat (Hersteller: Fa. Solutia). In obiger Tabelle sind die Trockengewichte als Einwaage angegeben. | | | | | |
| Scotchpak® 1022 Release Liner ist ein Produkt der Fa. 3M, St. Paul, MN, USA. Saran-Hytrel® Backing ist ein Produkt der Fa. Bertek, St. Albans, VT, USA. | | | | | |

### Beispiel 2: Penetrationsstudien

Die Ergebnisse der Penetrationsstudien mit den entsprechenden fünf Formulierungen mit unterschiedlichen Gehalten an Hydroxytriendion sind in den folgenden Tabellen dargestellt.
Die Messungen wurden mit folgendem in-vitro-Diffusionstest durchgeführt:
Eine temperierte Franz-Durchflusszelle wird durch ein 2 cm² großes Stück excidierte Haut von haarlosen Mäusen in ein Donor- und ein Akzeptorkompartiment geteilt, wobei das Stratum corneum der Donorseite zugewandt ist. Eine 3%ige oder 5 %ige Lösung von Hydroxypropyl-β-cyclodextrin in Puffer wird mit Hilfe einer pneumatischen Pumpe aus einem temperierten Vorratsbehälter durch das Akzeptorkompartiment gepumpt und mit Hilfe eines Fraktionssammlers in Glasvials gesammelt, die in bestimmten Zeitabständen ausgetauscht werden.
   Auf der Donorseite werden die wirkstoffhaltigen Pflaster aufgeklebt.
   Der Gehalt an Wirkstoffen wird in den einzelnen Fraktionen mittels HPLC/UV bzw. GC/FID bestimmt.
   Der transdermale in-vitro-Hautfluss wird in ng/cm²/h angegeben:

| Formulierung 1: 1 % Hydroxytriendion | | | | | | | |
|---|---|---|---|---|---|---|---|
| [h] | 1 | 2 | 3 | 4 | MW | SD | CV |
| 1 | 2 | 1 | 0 | 0 | 1 | 1 | 86,6 % |
| 3 | 16 | 13 | 2 | 6 | 9 | 6 | 70,4 % |
| 5 | 55 | 36 | 11 | 27 | 32 | 18 | 57,2% |
| 10 | 110 | 57 | 45 | 72 | 71 | 28 | 39,8 % |
| 18 | 79 | 59 | 51 | 76 | 66 | 13 | 20,2% |
| 26 | 77 | 44 | 48 | 111 | 70 | 31 | 44,3 % |
| 34 | 56 | 32 | 47 | 52 | 47 | 11 | 22,6% |
| 42 | 62 | 26 | 40 | 51 | 45 | 15 | 33,9% |
| 50 | 40 | 20 | 39 | 42 | 35 | 10 | 29,0 % |

| Formulierung 2: 3 % Hydroxytriendion | | | | | | | |
|---|---|---|---|---|---|---|---|
| [h] | 1 | 2 | 3 | 4 | MW | SD | CV |
| 1 | 1 | 1 | 0 | 1 | 1 | 0 | 71,1 % |
| 3 | 20 | 24 | 4 | 16 | 16 | 9 | 54,3 % |
| 5 | 64 | 111 | 17 | 61 | 63 | 38 | 60,6 % |
| 10 | 118 | 171 | 109 | 207 | 151 | 46 | 30,6 % |
| 18 | 118 | 144 | 166 | 161 | 148 | 22 | 14,6 % |
| 26 | 106 | 147 | 197 | 158 | 152 | 37 | 24,5 % |
| 34 | 71 | 115 | 160 | 142 | 122 | 39 | 31,8 % |
| 42 | 54 | 114 | 120 | 116 | 101 | 31 | 30,9 % |
| 50 | 46 | 91 | 68 | 103 | 77 | 25 | 32,8 % |

| Formulierung 3: 5 % Hydroxytriendion | | | | | | | |
|---|---|---|---|---|---|---|---|
| [h] | 1 | 2 | 3 | 4 | MW | SD | CV |
| 1 | 2 | 1 | 1 | 2 | 1 | 1 | 67,3% |
| 3 | 33 | 18 | 13 | 24 | 22 | 8 | 38,3 % |
| 5 | 104 | 57 | 48 | 86 | 74 | 26 | 34,9 % |
| 10 | 177 | 212 | 302 | 251 | 236 | 54 | 22,8 % |
| 18 | 168 | 280 | 228 | 345 | 255 | 75 | 29,4 % |
| 26 | 168 | 393 | 231 | 393 | 296 | 115 | 38,7 % |
| 34 | 196 | 206 | 240 | 263 | 226 | 31 | 13,7 % |
| 42 | 152 | 162 | 182 | 180 | 169 | 15 | 8,7 % |
| 50 | 147 | 147 | 221 | 138 | 163 | 39 | 23,9 % |

| Formulierung 4: 10 % Hydroxytriendion | | | | | | | |
|---|---|---|---|---|---|---|---|
| [h] | 1 | 2 | 3 | 4 | MW | SD | CV |
| 1 | 0 | 5 | 3 | 1 | 2 | 2 | 87,9 % |
| 3 | 16 | 74 | 60 | 35 | 46 | 26 | 55,6 % |
| 5 | 71 | 239 | 192 | 123 | 156 | 74 | 47,6 % |
| 10 | 349 | 466 | 428 | 477 | 430 | 58 | 13,5 % |
| 18 | 451 | 262 | 494 | 291 | 375 | 115 | 30,8 % |
| 26 | 695 | 269 | 463 | 242 | 417 | 209 | 50,2 % |
| 34 | 387 | 272 | 294 | 209 | 290 | 74 | 25,5 % |
| 42 | 257 | 232 | 242 | 165 | 224 | 41 | 18,2 % |
| 50 | 321 | 102 | 145 | 147 | 179 | 97 | 54,3 % |

| Formulierung 5: 15 % Hydroxytriendion | | | | | | |
|---|---|---|---|---|---|---|
| h | 1 | 2 | 4 | MW | SD | CV |
| 1 | 2 | 5 | 5 | 4 | 2 | 49,7 % |
| 3 | 43 | 69 | 68 | 60 | 14 | 23,8 % |
| 5 | 213 | 182 | 187 | 194 | 17 | 8,6 % |
| 10 | 486 | 408 | 543 | 479 | 67 | 14,1 % |
| 18 | 374 | 404 | 276 | 352 | 67 | 19,0 % |
| 26 | 400 | 487 | 263 | 383 | 113 | 29,0 % |
| 34 | 386 | 218 | 220 | 275 | 96 | 35,1 % |
| 42 | 261 | 184 | 174 | 206 | 48 | 23,1 % |
| 50 | 271 | 176 | 160 | 202 | 60 | 29,6 % |

Die Mittelwerte und Standardabweichungen für die transdermalen in-vitro-Hautflüsse ergeben sich aus der nachfolgenden Tabelle II und sind in Fig. 2 graphisch dargestellt

**Tabelle II**

| Mittelwerte: | | | | | |
|---|---|---|---|---|---|
| [h] | 1 % | 3% | 5% | 10% | 15% |
| 1 | 1 | 1 | 1 | 2 | 4 |
| 3 | 9 | 16 | 22 | 46 | 60 |
| 5 | 32 | 63 | 74 | 156 | 194 |
| 10 | 71 | 151 | 236 | 430 | 479 |
| 18 | 66 | 148 | 255 | 375 | 352 |
| 26 | 70 | 152 | 296 | 417 | 383 |
| 34 | 47 | 122 | 226 | 290 | 275 |
| 42 | 45 | 101 | 169 | 224 | 206 |
| 50 | 35 | 77 | 163 | 179 | 202 |

| Standardabweichungen: | | | | | |
|---|---|---|---|---|---|
| [h] | 1% | 3% | 5% | 10% | 15% |
| 1 | 1 | 0 | 1 | 2 | 2 |
| 3 | 6 | 9 | 8 | 26 | 14 |
| 5 | 18 | 38 | 26 | 74 | 17 |
| 10 | 28 | 46 | 54 | 58 | 67 |
| 18 | 13 | 22 | 75 | 115 | 67 |
| 26 | 31 | 37 | 115 | 209 | 113 |
| 34 | 11 | 39 | 31 | 74 | 96 |
| 42 | 15 | 31 | 15 | 41 | 48 |
| 50 | 10 | 25 | 39 | 97 | 60 |

### Beispiel 3: Vergleichsversuch zwischen Gestoden und Hydroxytriendion

Zum Vergleich wurden dieselben Messungen der transdermalen in-vitro-Hautflüsse - bei ansonsten gleicher Formulierung - mit Gestoden durchgeführt. Die entsprechenden Ergebnisse ergeben sich aus den nachfolgenden Tabellen.

| Transdermale in-vitro-Hautfluss in ng/cm²/h (1 % Gew.-% Gestoden) | | | | | | | |
|---|---|---|---|---|---|---|---|
| [h] | 1 | 2 | 3 | 4 | MW | SD | CV |
| 1 | 0 | 0 | 0 | 0 | 0 | 0 | - |
| 3 | 1 | 4 | 2 | 5 | 3 | 2 | 64,3 % |
| 5 | 5 | 31 | 14 | 18 | 17 | 11 | 64,3 % |
| 10 | 39 | 72 | 80 | 58 | 62 | 18 | 28,6 % |
| 18 | 77 | 46 | 110 | 36 | 67 | 34 | 49,8 % |
| 26 | 120 | 45 | 111 | 33 | 77 | 45 | 57,8 % |
| 34 | 50 | 39 | 83 | 31 | 51 | 23 | 44,6 % |
| 42 | 47 | 33 | 74 | 25 | 45 | 22 | 49,0 % |
| 50 | 37 | 27 | 44 | 22 | 32 | 10 | 30,0 % |

Aus den Versuchsergebnissen (Fig. 2) ist ersichtlich, dass bei Einsatz des Gestagens Hydroxytriendion ein deutlich höherer transdermaler in-vitro-Hautfluss als bei Einsatz von Gestoden zu beobachten ist, der auf die wesentlich höhere Beladbarkeit der Matrix-TDS mit Hydroxytriendion zurückzuführen ist.

## Patentansprüche

1. Transdermalsystem **gekennzeichnet durch** einen Gehalt an (21S)-21-Hydroxy-21-methyl-14,17-ethano-19-norpregna-4,9,15-trien-3,20-dion.

2. Transdermalsystem nach Anspruch 1, **dadurch gekennzeichnet, dass** (21S)-21-Hydroxy-21-methyl-14,17-ethano-19-norpregna-4,9,15-trien-3,20-dion in einer Matrix vorliegt.

3. Transdermalsystem nach Anspruch 2, **dadurch gekennzeichnet, dass** die Matrix Polyacrylatkleber umfasst.

4. Transdermalsystem nach Anspruch 3, **dadurch gekennzeichnet, dass** der Polyacrylatkleber ein Copolymer von zumindest 2 der folgenden Monomere ist: 2-Ethylhexylhexylacrylat, Hydroxyethylhexylacrylat, Vinylacetat, Vinylpyrrolidon.

5. Transdermalsystem nach Anspruch 4, **dadurch gekennzeichnet, dass** der Polyacrylatkleber ein Copolymer aus 2-Ethylhexylacrylat und Hydroxyethylacrylat (Gelva® ) oder ein Copolymer dieser Monomere mit Vinylacetat und 2-Ethyl-hexylacrylat-N-vinyl-2-pyrrolidon (TSR® -Kleber der Firma Sekisui) ist

6. Transdermalsystem nach einem der Ansprüche 2 bis 5, **gekennzeichnet durch** einen Gehalt von 0,1 bis 20 Gew.-%, vorzugsweise 1 bis 15% (21 S)-21-Hydroxy-21-methyl-14,17-ethano-19-norpregna-4,9,15-trien-3,20-dion in der Matrix.

7. Transdermalsystem nach einem der Ansprüche 2 bis 6, **dadurch gekennzeichnet, dass** die Matrix wenigstens einen Kristallisationsinhibitor umfasst.

8. Transdermalsystem nach Anspruch 7, **dadurch gekennzeichnet, dass** die Matrix wenigstens ein N-Vinyllactam-Polymer wie N-Vinyl-1-Azacycloheptan-2-on-Homopolymer, N-Vinyl-piperidin-2-on-Homopolymer, Polymere des Vinylpyrrolidons wie Polyvidon (Kollidon® ), oder Co-Polymere des Vinylpyrrolidons mit Vinylacetat (Copovidone) umfasst.

9. Transdermalsystem nach einem der Ansprüche 1 bis 8, **gekennzeichnet durch** einen zusätzlichen Gehalt an wenigstens einem der folgenden Penetrationsverstärker:
ein- oder mehrwertige Alkohole wie Ethanol, 1,2-Propandiol oder Benzylalkohol; gesättigte oder ungesättigte Fettalkohole mit 8 bis 18 Kohlenstoffatomen wie Laurylalkohol oder Cetylalkohol; Kohlenwasserstoffe wie Mineralöl; gesättigte und ungesättigte Fettsäuren mit 8 bis 18 Kohlenstoffatomen wie Stearinsäure oder Ölsäure; Fettsäureester mit bis zu 24 Kohlenstoffatomen oder Dicarbonsäurediester mit bis zu 24 Kohlenstoffatomen wie die Methylester, Ethylester, Isopropylester, Butylester, sek.-Butylester, Isobutylester, tert.-Butylester oder Monoglycerinsäureester der Essigsäure, Capronsäure, Laurinsäure, Myristinsäure, Stearinsäure und Palmitinsäure, Phosphatidderivate, wie Lecithin, Terpene, Harnstoff und seine Derivate oder Ether wie Dimethylisosorbid und Diethylenglycolmonoethylether.

10. Transdermalsystem nach Anspruch 9, **gekennzeichnet durch** einen Gehalt an wenigstens einem der folgenden Penetrationsverstärker:
Laurylalkohol, 1,2-Propandiol, die Methylester und insbesondere die Isopropylester der Myristinsäure oder Ölsäure, Diisopropyladipat und Diisopropylsebacat, Laurinsäure und Ölsäure, sowie deren Gemische.

11. Transdermalsystem nach einem der vorangehenden Ansprüche, **gekennzeichnet durch** einen zusätzlichen Gehalt an wenigstens einem Estrogen wie Estradiol, Estriol, Ethinylestradiol, deren Derivate wie beispielsweise Mono- und Di-ester wie Estradiol-3,17ß-diproprionat.
